# EUROPEAN PATENT APPLICATION

(11) **EP 2 856 945 A1**
(43) Date of publication of application: **08.04.2015**
(21) Application number: 13800084.9
(22) Date of filing: 02.05.2013
(51) Int. Cl.: A61B 8/06, A61B 8/08

(54) **OPTOACOUSTIC DIAGNOSIS APPARATUS, METHOD, PROGARAM AND RECORDING MEDIUM**

(30) Priority: 04.06.2012 JP 2012127021
(71) Applicant: Advantest Corporation, Tokyo 179-0071 (JP)
(72) Inventor: KAWAGUCHI, Yasushi, Tokyo 179-0071 (JP); IDA, Taiichiro, Tokyo 179-0071 (JP)
(74) Representative: Hess, Peter K. G.
(86) International application number: PCT/JP2013/063233
(87) International publication number: WO 2013/183401

(57) **Abstract**

A photoacoustic diagnosis device that diagnoses a state of a skin of a human body, includes a pulsed light source, an electric signal converter, a blood distribution obtaining section, and a diagnosis section. The pulsed light source generates a pulsed light. The electric signal converter receives a photoacoustic wave generated at the skin by the pulsed light and converts the photoacoustic wave into an electric signal. The blood distribution obtaining section obtains distribution of blood in the skin based on the electric signal. The diagnosis section diagnoses a state of the skin based on a result obtained by the blood distribution obtaining section.

## Description

### FIELD OF THE INVENTION

The present invention relates to diagnosis of a burn or the like, using a photoacoustic wave.

### BACKGROUND ART

Photoacoustic sensors are conventionally known to measure a photoacoustic signal generated by irradiating an object to be measured (e.g. biological object) with pulsed light (see, for example, Patent Document 1 (Japanese Unexamined Patent Publication No. 2011-229660)).

### SUMMARY OF THE INVENTION

Accordingly, it is an object of the present invention to diagnose a human body using a photoacoustic signal obtained by a photoacoustic sensor.

According to the present invention, a photoacoustic diagnosis device that diagnoses a state of a skin of a human body, includes: a pulsed light source that generates a pulsed light; an electric signal converter that receives a photoacoustic wave generated at the skin by the pulsed light and converts the photoacoustic wave into an electric signal; a blood distribution obtaining section that obtains distribution of blood in the skin based on the electric signal; and a diagnosis section that diagnoses a state of the skin based on a result obtained by the blood distribution obtaining section.

According to the thus constructed photoacoustic diagnosis device, a photoacoustic diagnosis device that diagnoses a state of a skin of a human body can be provided. A pulsed light source generates a pulsed light. An electric signal converter receives a photoacoustic wave generated at the skin by the pulsed light and converts the photoacoustic wave into an electric signal. A blood distribution obtaining section obtains distribution of blood in the skin based on the electric signal. A diagnosis section diagnoses a state of the skin based on a result obtained by the blood distribution obtaining section.

According to the photoacoustic diagnosis device of the present invention, the diagnosis section may diagnose the state of the skin based on a distribution of blood in an epidermis or a dermis of the skin.

According to the photoacoustic diagnosis device of the present invention, the diagnosis section may diagnose a burn based on the distribution of the blood in the epidermis or dermis of the skin.

According to the photoacoustic diagnosis device of the present invention, the diagnosis section may diagnose gangrene based on the distribution of the blood in the epidermis or dermis of the skin.

According to the photoacoustic diagnosis device of the present invention, the diagnosis section may diagnose decubitus based on the distribution of the blood in the epidermis or dermis of the skin.

According to the photoacoustic diagnosis device of the present invention, the pulsed light may have two or more kinds of wavelength components, and the diagnosis section may diagnose a state of a substance existing in the skin based on the electric signal corresponding to each wavelength component.

According to the photoacoustic diagnosis device of the present invention, the substances existing in the skin may be oxygenated hemoglobin and deoxygenated hemoglobin.

According to the present invention, the photoacoustic diagnosis device may further include a display section that displays a result obtained by the blood distribution obtaining section.

According to the photoacoustic diagnosis device of the present invention, the pulsed light source may be a fiber laser.

The present invention is a photoacoustic diagnosis method with using a photoacoustic diagnosis device that diagnoses a state of a skin of a human body, having a pulsed light source that generates a pulsed light, and an electric signal converter that receives a photoacoustic wave generated at the skin by the pulsed light and converts the photoacoustic wave into an electric signal, the method including: a blood distribution obtaining step that obtains distribution of blood in the skin based on the electric signal; and a diagnosis step that diagnoses a state of the skin based on a result obtained by the blood distribution obtaining step.

The present invention is a program of instructions for execution by a computer to perform a photoacoustic diagnosis process with using a photoacoustic diagnosis device that diagnoses a state of a skin of a human body, having a pulsed light source that generates a pulsed light, and an electric signal converter that receives a photoacoustic wave generated at the skin by the pulsed light and converts the photoacoustic wave into an electric signal, the process including: a blood distribution obtaining step that obtains distribution of blood in the skin based on the electric signal; and a diagnosis step that diagnoses a state of the skin based on a result obtained by the blood distribution obtaining step.

The present invention is a computer-readable medium having a program of instructions for execution by a computer to perform a photoacoustic diagnosis process with using a photoacoustic diagnosis device that diagnoses a state of a skin of a human body, having a pulsed light source that generates a pulsed light, and an electric signal converter that receives a photoacoustic wave generated at the skin by the pulsed light and converts the photoacoustic wave into an electric signal, the process including: a blood distribution obtaining step that obtains distribution of blood in the skin based on the electric signal; and a diagnosis step that diagnoses a state of the skin based on a result obtained by the blood distribution obtaining step.

### BRIEF DESCRIPTION OF DRAWINGS

Fig. 1 shows a functional block diagram of the configuration of a photoacoustic diagnosis device 1 according to one embodiment of the present invention; and
Fig. 2 shows an example of display of the image display section 20 obtained when a skin 2 gets burned (second degree) (see Fig. 2(a)), and an example of display of the image display section 20 obtained when the skin 2 gets burned (third degree) (see Fig. 2(b)).

### Modes for Carrying out the Invention

In the following, preferred embodiment of the present invention will be described with reference to the accompanying drawings.

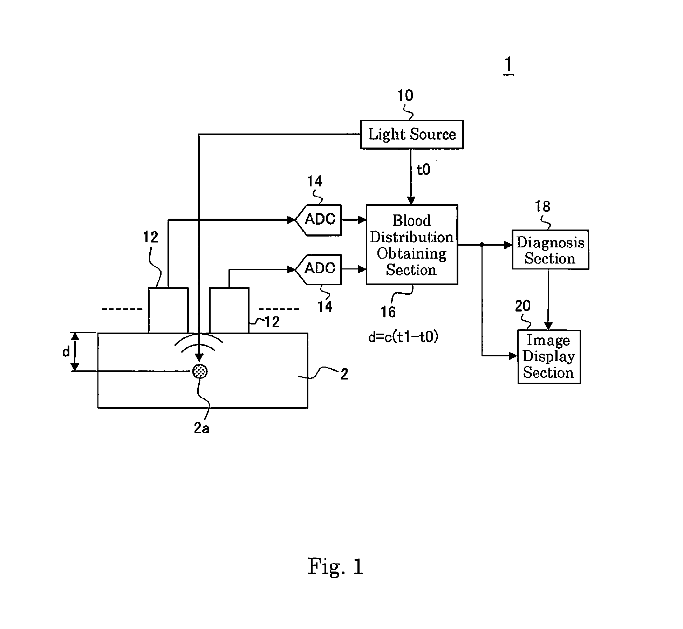

Fig. 1 shows a functional block diagram of the configuration of a photoacoustic diagnosis device 1 according to one embodiment of the present invention. The photoacoustic diagnosis device 1 includes a light source 10, ultrasonic transducers (electric signal converters) 12, A/D converters 14, a blood distribution obtaining section 16, a diagnosis section 18, and an image display section 20.

The photoacoustic diagnosis device 1 is adapted to diagnose the state of a skin 2 of the human body.

The light source 10 generates pulsed light. The wavelength of the pulsed light may have one type of wavelength component, but may have two or more types of wavelength components. The light source 10 can reduce the size of the photoacoustic diagnosis device 1 by using a fiber laser (for example, an optical fiber-ring laser).

The skin 2 has a blood vessel 2a. When receiving the pulsed light, the blood vessel 2a generates a photoacoustic wave. The ultrasonic transducer 12 receives the photoacoustic wave and converts the wave into an electric signal (for example, in the form of a voltage). The ultrasonic transducer 12 is, for example, a piezoelectric element. A plurality of the ultrasonic transducers 12 is preferably provided.

The A/D converters 14 are connected to the respective ultrasonic transducers 12, and convert the electric signal (which is an analog signal) output from each ultrasonic transducer 12 into a digital signal.

The blood distribution obtaining section 16 receives the electric signal via the A/D converters 14, and obtains the distribution of blood in the skin 2 based on the electric signal received.

The depth of the blood is measured to determine the distribution of the blood. The term "depth of blood" as used herein means a depth d of a blood (for example, the blood existing in the blood vessel 2a) with respect to the surface of the skin 2. The depth d can be determined in the form of, for example, c(t1 - t0), where c is a sound speed (speed of a photoacoustic wave propagating in the skin 2), t1 is the time when the photoacoustic wave reaches the ultrasonic transducer 12, and t0 is the time when the pulsed light is generated. The blood distribution obtaining section 16 receives data on the time t0 of generation of the pulsed light from the light source 10.

The diagnosis section 18 diagnoses the state of the skin 2 based on the result obtained by the blood distribution obtaining section 16. The image display section 20 displays the result obtained by the blood distribution obtaining section 16.

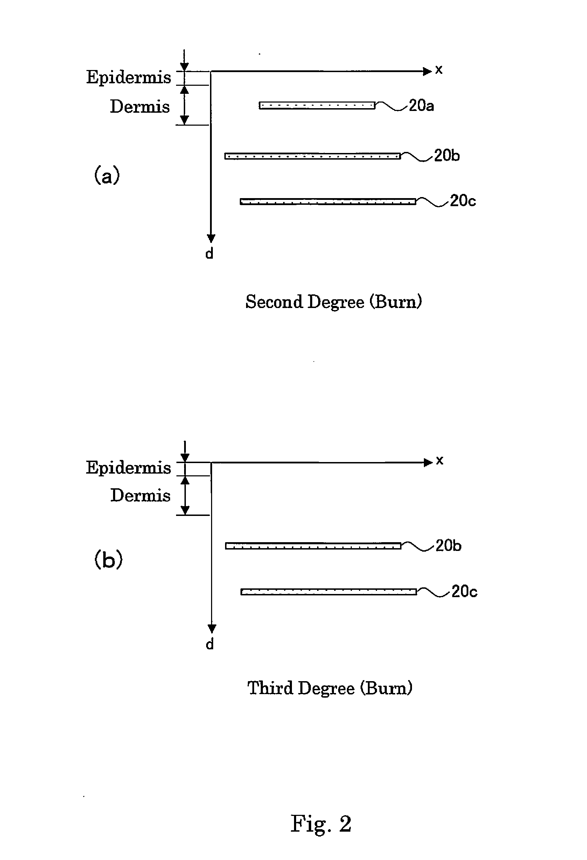

Fig. 2 shows an example of display of the image display section 20 obtained when a skin 2 gets burned (second degree) (see Fig. 2(a)), and an example of display of the image display section 20 obtained when the skin 2 gets burned (third degree) (see Fig. 2(b)).

Burns are classified into three degrees according to the depth which the burn reached, namely, the first degree (damage to a horny layer of an epidermis), the second degree, and the third degree (damage to the whole dermis layer and subcutaneous tissue). While the epidermis has no bloodstream, the dermis has capillary blood vessels with the bloodstream. In the burn of second degree, at least a part of the dermis escapes being burned, and the bloodstream remains in the dermis. On the other hand, in the burn of third degree, the entire dermis gets burned, and as a result, no bloodstream remains in the dermis.

Referring to Fig. 2, a description will be given of the principle of diagnosis of the burn performed by the diagnosis section 18. In Fig. 2, reference sign "d" indicates a depth of the burn, and "x" indicates an axis of any straight line on the surface of the skin. The depths of the epidermis and the dermis from the surface of the skin are substantially well known, which are illustrated in Fig. 2.

Referring to Fig. 2(a), the image display section 20 displays bloods 20a, 20b, and 20c. Since the blood 20a exists in the depth of the dermis, the bloodstream can also be supposed to exist. It is found that the burn belongs to the second degree. It is noted that the skin 2 could also be determined to get the burn of first degree or no burn. However, such a case can be easily identified from the appearance of the skin 2.

Referring to Fig. 2(b), the image display section 20 displays the bloods 20b, and 20c. Since no blood exists in the depth of the dermis, the bloodstream can also be supposed not to exist. It is found that the burn belongs to the third degree.

Although the diagnosis of the burn has been described above, the present invention is not limited thereto. The diagnosis section 18 can be used for any other diagnosis of the state of the skin. For example, gangrene (for example, caused by the diabetes) and decubitus (or bedsore) can also be diagnosed. That is, the presence or absence of the bloodstream in the dermis depends on whether or not the entire dermis is damaged or not, or whether at least a part of the dermis escapes being damaged or not. For this reason, the state of damage to the dermis (corresponding to the progressed state of the gangrene and decubitus) can be diagnosed by the presence or absence of the blood in the dermis. Alternatively, the extent of the exposure to chemicals, the distribution of medical agent, and the degree of penetration of cosmetics can be measured.

As mentioned above, the wavelength of the pulsed light may have two or more kinds of wavelength components. In this case, the diagnosis section 18 diagnoses the state of a substance existing in the skin 2 based on the electric signal corresponding to each wavelength component. The substances existing in the skin are, for example, oxygenated hemoglobin and deoxygenated hemoglobin.

The wavelengths of the pulsed light include a wavelength λa that can be more easily absorbed in the oxygenated hemoglobin than in the deoxygenated hemoglobin, and a wavelength λb that can be more easily absorbed in the deoxygenated hemoglobin than in the oxygenated hemoglobin.

When the strength of an electric signal corresponding to a photoacoustic signal generated due to the wavelength λa is higher than that of another electric signal corresponding to a photoacoustic signal generated due to the wavelength λb, the blood can be diagnosed to contain the oxygenated hemoglobin in a larger amount than the deoxygenated hemoglobin.

In contrast, when the strength of an electric signal corresponding to a photoacoustic signal generated due to the wavelength λb is higher than that of another electric signal corresponding to a photoacoustic signal generated due to the wavelength λa, the blood can be diagnosed to contain the deoxygenated hemoglobin in a larger amount than the oxygenated hemoglobin.

Next, the operation of the embodiment in the invention will be described below.

First, a pulsed light from the light source 10 is applied to the skin 2 and reaches the blood vessel 2a. Then, the blood vessel 2a absorbs the pulsed light and is warmed and is then adiabatically expanded. Thus, the compression waves (photoacoustic waves) are output from the blood vessel 2a.

The photoacoustic wave reaches the ultrasonic transducers 12. The ultrasonic transducers 12 convert pressure caused by the photoacoustic wave into an electric signal (for example, in the form of a voltage). The voltage is converted into a digital signal by the A/D converters 14 and then fed to the blood distribution obtaining section 16.

The blood distribution obtaining section 16 gives the result obtained to the diagnosis section 18 and the image display section 20. The diagnosis section 18 performs diagnosis shown in Fig. 2 and sends the result of the diagnosis to the image display section 20. The image display section 20 displays the diagnosis result obtained by the diagnosis section 18 that is superimposed on the result obtained by the blood distribution obtaining section 16.

The photoacoustic diagnosis device 1 of the embodiment in the present invention can make a diagnosis of the skin 2 of the human body.

The embodiment described above can be implemented in the following way. A computer with a CPU, a hard disk, and a media (floppy (trademark) disk, CD-ROM, etc.) reader is adapted to read media that store therein programs for achieving the above-mentioned components, for example, the blood distribution obtaining section 16 and the diagnosis section 18. Then, the media read are installed in the hard disk. Even this method can achieve the above-mentioned functions.

## Claims

1. A photoacoustic diagnosis device that diagnoses a state of a skin of a human body, comprising:
a pulsed light source that generates a pulsed light;
an electric signal converter that receives a photoacoustic wave generated at the skin by the pulsed light and converts the photoacoustic wave into an electric signal;
a blood distribution obtaining section that obtains distribution of blood in the skin based on the electric signal; and
a diagnosis section that diagnoses a state of the skin based on a result obtained by the blood distribution obtaining section.

2. The photoacoustic diagnosis device according to claim 1, wherein the diagnosis section diagnoses the state of the skin based on a distribution of blood in an epidermis or a dermis of the skin.

3. The photoacoustic diagnosis device according to claim 2, wherein the diagnosis section diagnoses a burn based on the distribution of the blood in the epidermis or dermis of the skin.

4. The photoacoustic diagnosis device according to claim 2, wherein the diagnosis section diagnoses gangrene based on the distribution of the blood in the epidermis or dermis of the skin.

5. The photoacoustic diagnosis device according to claim 2, wherein the diagnosis section diagnoses decubitus based on the distribution of the blood in the epidermis or dermis of the skin.

6. The photoacoustic diagnosis device according to claim 1, wherein the pulsed light has two or more kinds of wavelength components, and the diagnosis section diagnoses a state of a substance existing in the skin based on the electric signal corresponding to each wavelength component.

7. The photoacoustic diagnosis device according to claim 6, wherein the substances existing in the skin are oxygenated hemoglobin and deoxygenated hemoglobin.

8. The photoacoustic diagnosis device according to claim 1, further comprising a display section that displays a result obtained by the blood distribution obtaining section.

9. The photoacoustic diagnosis device according to claim 1, wherein the pulsed light source is a fiber laser.

10. A photoacoustic diagnosis method with using a photoacoustic diagnosis device that diagnoses a state of a skin of a human body, having a pulsed light source that generates a pulsed light, and an electric signal converter that receives a photoacoustic wave generated at the skin by the pulsed light and converts the photoacoustic wave into an electric signal, said method comprising:
a blood distribution obtaining step that obtains distribution of blood in the skin based on the electric signal; and
a diagnosis step that diagnoses a state of the skin based on a result obtained by the blood distribution obtaining step.

11. A program of instructions for execution by a computer to perform a photoacoustic diagnosis process with using a photoacoustic diagnosis device that diagnoses a state of a skin of a human body, having a pulsed light source that generates a pulsed light, and an electric signal converter that receives a photoacoustic wave generated at the skin by the pulsed light and converts the photoacoustic wave into an electric signal, said process comprising:
a blood distribution obtaining step that obtains distribution of blood in the skin based on the electric signal; and
a diagnosis step that diagnoses a state of the skin based on a result obtained by the blood distribution obtaining step.

12. A computer-readable medium having a program of instructions for execution by a computer to perform a photoacoustic diagnosis process with using a photoacoustic diagnosis device that diagnoses a state of a skin of a human body, having a pulsed light source that generates a pulsed light, and an electric signal converter that receives a photoacoustic wave generated at the skin by the pulsed light and converts the photoacoustic wave into an electric signal, said process comprising:
a blood distribution obtaining step that obtains distribution of blood in the skin based on the electric signal; and
a diagnosis step that diagnoses a state of the skin based on a result obtained by the blood distribution obtaining step.
